# EUROPÄISCHE PATENTANMELDUNG

(11) **EP 3 510 918 A1**
(43) Veröffentlichungstag der Anmeldung: **17.07.2019**
(21) Anmeldenummer: 18151145.2
(22) Anmeldetag: 11.01.2018
(51) Int. Cl.: A61B 5/00, A61B 5/113

(54) **ATMUNGSGETRIGGERTE AUFNAHME VON MEDIZINISCHEN ROHDATEN**

(71) Anmelder: Siemens Healthcare GmbH, 91052 Erlangen (DE)
(72) Erfinder: Kartäusch, Ralf, 91058 Erlangen (DE)

(57) **Zusammenfassung**

Die Erfindung betrifft ein Verfahren zu einer atmungsgetriggerten Aufnahme von medizinischen Rohdaten eines Patienten gemäß den folgenden Verfahrensschritten:
- Erfassen eines zeitaufgelösten Atemsignals von zumindest einem Atemzyklus des Patienten,
- Vorhersage eines zukünftigen Atemsignals des Patienten basierend auf dem zeitaufgelösten Atemsignal,
- Triggerung einer Aufnahme von medizinischen Rohdaten des Patienten basierend auf dem vorhergesagten zukünftigen Atemsignal.

## Beschreibung

Die Erfindung betrifft ein Verfahren, ein medizinisches Bildgebungsgerät, ein Computerprogrammprodukt sowie einen elektronisch lesbaren Datenträger zu einer atmungsgetriggerten Aufnahme von medizinischen Rohdaten.

In einem Magnetresonanzgerät wird üblicherweise der zu untersuchende Körper eines Untersuchungsobjektes, insbesondere eines Patienten, mit Hilfe eines Magneten einem relativ hohen statischen Magnetfeld, beispielsweise von 1,5 oder 3 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse (HF-Pulse), beispielsweise Anregungspulse, ausgesendet, was dazu führt, dass die Kernspins bestimmter, durch diese HF-Pulse resonant angeregter Atome um einen definierten Flipwinkel gegenüber den Magnetfeldlinien des statischen Magnetfelds verkippt werden. Bei der Relaxation der Kernspins werden HF-Signale, so genannte Magnetresonanz-Signale, abgestrahlt, die mittels einer Empfangseinheit umfassend Spuleneinheiten, welche als HF-Antennen ausgebildet sind, empfangen und dann weiterverarbeitet werden. So werden medizinische Rohdaten aufgenommen, welche schließlich zu den gewünschten Bilddaten rekonstruiert werden können.

Während der Aufnahme der medizinischen Rohdaten beeinflusst eine Bewegung des Untersuchungsobjektes die Rohdaten und die daraus zu rekonstruierenden Bilddaten typischerweise negativ. Physiologische Bewegungen, beispielsweise aufgrund von Atmung und/oder eines schlagenden Herzens sind nicht und/oder nur schwer vermeidbar. DE102014207124 A1 beschreibt eine Vorrichtung zur Erfassung einer Bewegung eines Patienten mittels Radar. DE102014209488 A1 beschreibt ein Verfahren zur Vermessung eines Atemvorgangs eines Untersuchungsobjektes basierend auf Reflektionseigenschaften eines unterhalb des Untersuchungsobjektes angeordneten Spulenelements. DE102014208537 A1 beschreibt eine Vorrichtung zur Erfassung einer Bewegung eines Patienten mittels einer vom Magnetresonanzgerät umfassten Hochfrequenzantenneneinheit.

Beeinflusst eine physiologische Bewegung den Untersuchungsbereich des Untersuchungsobjektes, so ist eine Anpassung und/oder Synchronisation der Aufnahme der Rohdaten mit der physiologischen Bewegung vorteilhaft. Da die Aufnahme der Rohdaten mittels eines Magnetresonanzgerätes mehrere Minuten dauern kann, ist insbesondere in der Magnetresonanzbildgebung eine Synchronisation der Aufnahme der Rohdaten mit der Atmung des Patienten vorteilhaft. Hierfür sind eine Überwachung der Atmung des Patienten und eine entsprechende Triggerung der Aufnahme der medizinischen Rohdaten erforderlich. Die Triggerung kann einen Beginn einer Aufnahme von medizinischen Rohdaten und/oder ein Kommando an den Patienten zum Anhalten der Atmung umfassen.

Herkömmlich erfolgt die Triggerung anhand eines Schwellwertes für ein Atemsignal des Patienten. Der Schwellwert für die Triggerung kann beispielsweise anhand einer statistischen Auswertung vorheriger Atemzyklen des Patienten ermittelt werden. Bei Überschreitung und/oder Unterschreitung des Schwellwertes kann die Triggerung der Aufnahme ausgelöst werden. Dies funktioniert insbesondere bei ungestörten periodischen Atemzyklen. Überlagert sich der Atmung eine weitere Bewegung des Patienten und/oder ändert der Patient die Ausatemtiefe, so wird das Atemsignal typischerweise derart moduliert, dass der Schwellwert für die Triggerung nicht mehr gültig ist. Dadurch werden häufig Zeitpunkte für die Triggerung nicht erkannt. Dies trifft typischerweise so lange zu, bis entweder der Patient die ursprüngliche Ausatemtiefe erreicht und/oder die statistische Auswertung vorheriger Atemzyklen zur Bestimmung des Schwellwertes die Atemzyklen mit der neuen Ausatemtiefe umfasst. Dadurch kann sich die Aufnahmedauer verlängern und/oder die Aufnahme der medizinischen Rohdaten abgebrochen werden.

Der Erfindung liegt die Aufgabe zugrunde, ein besonders robustes Verfahren zu einer atmungsgetriggerten Aufnahme von medizinischen Rohdaten anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zu einer atmungsgetriggerten Aufnahme von medizinischen Rohdaten eines Patienten sieht die folgenden Verfahrensschritte vor:
- Erfassen eines zeitaufgelösten Atemsignals von zumindest einem Atemzyklus des Patienten,
- Vorhersage eines zukünftigen Atemsignals des Patienten basierend auf dem zeitaufgelösten Atemsignal,
- Triggerung einer Aufnahme von medizinischen Rohdaten des Patienten basierend auf dem vorhergesagten zukünftigen Atemsignal.

Bei einer atmungsgetriggerten Aufnahme medizinischer Rohdaten erfolgt eine zeitliche Abstimmung einer Akquisition medizinischer Rohdaten mit der Atmung des Patienten. Dabei können Zeitfenster für die Aufnahme medizinischer Rohdaten abhängig vom zeitlichen Verlauf der Atmung des Patienten vorgegeben werden. Zusätzlich und/oder alternativ kann abhängig vom zeitlichen Verlauf der Atmung des Patienten ein Zeitpunkt bestimmt werden, zu welchem Zeitpunkt der Patient den Atem anhalten soll und/oder ein entsprechendes Signal an den Patienten abgegeben wird.

Die Atmung eines Patienten erfolgt typischerweise zyklisch. Dabei umfasst die Atmung eines Patienten typischerweise Einatmung und Ausatmung. Ein Atemzyklus umfasst typischerweise eine Einatmung und eine Ausatmung. Ein Atemzyklus umfasst typischerweise genau eine Einatmung und genau eine Ausatmung. Ein Atemzyklus kann dabei zu einem beliebigen Zeitpunkt während der Atmung beginnen. Ein Atemzyklus kann demnach auch während einer Einatmung und/oder einer Ausatmung beginnen. Ein Atemzyklus beginnt vorzugsweise zu Beginn einer Einatmung oder einer Ausatmung. Ein Atemzyklus ist vorzugsweise zeitaufgelöst und/oder umfasst eine Dauer für die Einatmung und die Ausatmung.

Ein Atemsignal eines Patienten umfasst typischerweise ein Maß für die Menge an Luft in den Atemwegen des Patienten. Ein Atemsignal kann eine Position und/oder Bewegung des Patienten, insbesondere dessen Brustkorb und/oder dessen Oberkörper, umfassen und/oder aus einer derartigen Position und/oder Bewegung extrahiert werden. Das Atemsignal des Patienten kann beispielsweise mittels eines Sensors erfasst werden. Der Sensor kann beispielsweise am Oberkörper und/oder an den Atemwegen des Patienten positioniert sein. Das Atemsignal des Patienten kann auch bildbasiert und/oder mittels einer Kamera erfasst werden. Das Atemsignal kann anhand zeitaufgelöster medizinischer Bilddaten erfasst werden, welche medizinischen Bilddaten mit dem gleichen medizinischen Bildgebungsgerät wie die medizinischen Rohdaten erzeugt werden. Das erfasste Atemsignal des Patienten ist zeitaufgelöst. Das Erfassen des zeitaufgelösten Atemsignals des Patienten kann auch ein Bereitstellen und/oder Verarbeiten von Daten umfassen, welche Daten das zeitaufgelöste Atemsignal umfassen und/oder aus welchen Daten das zeitaufgelöste Atemsignal extrahiert werden kann. Das zeitaufgelöste Atemsignal umfasst vorzugsweise ein zeitaufgelöstes Atemsignal von zumindest einem Atemzyklus des Patienten. Das erfasste zeitaufgelöste Atemsignal umfasst typischerweise Atemsignal zumindest eines vergangenen Atemzyklus. Das erfasste zeitaufgelöste Atemsignal umfasst typischerweise Atemsignal des zuletzt vergangenen Atemzyklus.

Die Atmung des Patienten wird vorzugsweise kontinuierlich überwacht und/oder dessen Atemsignal wird kontinuierlich erfasst. Basierend auf dem kontinuierlich erfassten Atemsignal wird vorzugsweise nach Erfassen eines Atemsignals eines ersten Atemzyklus ein zukünftiges Atemsignals eines zweiten Atemzyklus vorhergesagt. Das Atemsignal des ersten Atemzyklus wird vorzugsweise vom kontinuierlich erfassten Atemsignal umfasst und entspricht vorzugsweise dem Atemsignal des zeitlich betrachtet letzten Atemzyklus des erfasste Atemsignals. Der zweite Atemzyklus ist zeitlich betrachtet vorzugsweise nach dem ersten Atemzyklus. Der zeitliche Abstand des ersten Atemzyklus und des zweiten Atemzyklus umfasst insbesondere maximal drei Atemzyklen, bevorzugt maximal zwei Atemzyklen, besonders bevorzugt maximal einen Atemzyklus. Der zweite Atemzyklus kann auch direkt an den ersten Atemzyklus anschließen.

Die Erfindung sieht vor, dass ein zukünftiges Atemsignal des Patienten basierend auf dem zeitaufgelösten Atemsignal ermittelt wird. Hierbei wird ein zukünftiges Atemsignal zu zumindest einem Zeitpunkt eines zukünftigen, insbesondere eines folgenden, Atemzyklus ermittelt. Vorzugsweise ist das zukünftige Atemsignal zeitaufgelöst für zumindest einen zukünftigen Atemzyklus. Das zukünftige Atemsignal wird vorzugsweise für zumindest einen Zeitpunkt vorhergesagt, welcher Zeitpunkt eine bekannte Funktion und/oder Atemposition beschreibt, wie beispielsweise einen Beginn eines Einatmens. Anhand dieses Zeitpunkts kann die Triggerung der Aufnahme von medizinischen Rohdaten des Patienten erfolgen. Bei der Vorhersage des zukünftigen Atemsignals wird vorzugsweise das zeitaufgelöste Atemsignal des zumindest einen zeitlich vorangehenden Atemzyklus des Patienten berücksichtigt. Vorzugsweise umfasst das bei der Vorhersage berücksichtigte zeitaufgelöste Atemsignal ein Atemsignal des zuletzt vergangenen Atemzyklus, insbesondere der drei zuletzt vergangenen Atemzyklen, besonders bevorzugt der sechs zuletzt vergangenen Atemzyklen. Dadurch kann die Triggerung instantan an eine Änderung der Atmung des Patienten angepasst werden. Es kann auf ein Abwarten einiger Atemzyklen des Patienten vor Anpassung der Triggerung, wie es beispielsweise bei statistischen Verfahren und/oder bei der Verwendung eines Schwellwertes erforderlich ist, verzichtet werden.

Das erfindungsgemäße Verfahren ermöglicht eine Vorhersage eines Atemsignals, wodurch prospektiv eine Atmung, insbesondere ein Atmungsverhalten, eines Patienten bei der Aufnahme von medizinischen Rohdaten berücksichtigt werden kann. Insbesondere kann gemäß dieser Erfindung ein Zeitpunkt für die Triggerung besonders robust bestimmt werden. Dies ermöglicht eine spontane und dynamische Berücksichtigung einer unregelmäßigen Atmung und/oder von nicht-periodischen Bewegungen des Patienten. Dadurch kann vorzugsweise jeder Atemzyklus zur Aufnahme von medizinischen Rohdaten verwendet werden, wodurch die Dauer der Aufnahme aller medizinischen Rohdaten verkürzt werden kann. Bei unregelmäßiger Atmung muss nicht auf eine Aufnahme medizinischer Rohdaten verzichtet werden. Ebenso ist ein Verwerfen derartig aufgenommener medizinischer Rohdaten nicht erforderlich, da anhand des vorhergesagten zukünftigen Atemsignals die Aufnahme der medizinischen Rohdaten an eine mögliche Bewegung aufgrund des zukünftigen Atemsignals angepasst werden kann. Die Qualität der medizinischen Rohdaten und damit die Qualität der aus den medizinischen Rohdaten rekonstruierbaren Bilddaten kann verbessert werden.

Eine Ausführungsform des Verfahrens sieht vor, dass die Vorhersage des zukünftigen Atemsignals basierend auf einem künstlichen neuronalen Netz erfolgt. Eine Eingabe des künstlichen neuronalen Netzes kann das erfasste zeitaufgelöste Atemsignal des Patienten sein. Eine Ausgabe des künstlichen neuronalen Netzes kann das zukünftige Atemsignal sein. Der Vorteil dieser Ausführungsform liegt darin, dass die Triggerung besonders robust erfolgt, da das erfasste zeitaufgelöste Atemsignal des Patienten im Rahmen der Vorhersage besonders genau analysiert werden kann.

Eine Ausführungsform des Verfahrens sieht vor, dass das künstliche neuronale Netz basierend auf Atemsignalen mehrerer Personen trainiert vorliegt. Das Training des künstlichen neuronalen Netzes erfolgt vorzugsweise anhand einer Datenbank umfassend Atemsignale mehrerer Atemzyklen mehrerer Personen. Das Training erfolgt vorzugsweise vor Ausführung des erfindungsgemäßen Verfahrens. Das Training erfolgt vorzugsweise einmalig. Das derartig trainierte künstliche neuronale Netz wird derart bereitgestellt, dass bei Vorhersage eines zukünftigen Atemsignals des Patienten darauf zugegriffen werden kann. Diese Ausführungsform ermöglicht eine schnelle Konvergenz des neuronalen Netzes bei Anwendung auf den Patienten und dessen erfasstes zeitaufgelöstes Atemsignal. Dadurch kann die Triggerung besonders zuverlässig ausgeführt werden.

Eine Ausführungsform des Verfahrens sieht vor, dass ein weiterer Verfahrensschritt ein Training des künstlichen neuronalen Netzes basierend auf dem zeitaufgelösten Atemsignal des zumindest einen Atemzyklus des Patienten umfasst und die Vorhersage des zukünftigen Atemsignals basierend auf dem trainierten künstlichen neuronalen Netz erfolgt. Gemäß dieser Ausführungsform erfolgt ein Training des künstlichen neuronalen Netzes auf den Patienten und insbesondere dessen Atmung. Dieser weitere Verfahrensschritt kann von der Vorhersage umfasst sein. Dadurch kann das zukünftige Atemsignal besonders genau vorhergesagt werden. Es ermöglicht eine besonders robuste Triggerung.

Eine Ausführungsform des Verfahrens sieht vor, dass das Training eine Prognose eines ersten Atemzyklus des zumindest einen Atemzyklus des Patienten und einen Vergleich des vorhergesagten Atemzyklus mit dem ersten Atemzyklus des zumindest einen Atemzyklus umfasst. Dieses Training wird vorzugsweise kontinuierlich ausgeführt. Vorzugsweise werden synchron zur Atmung des Patienten dessen Atemsignale umfassend den ersten Atemzyklus erfasst. Basierend auf Atemsignalen des Patienten ausschließlich des ersten Atemzyklus kann das Atemsignal und/oder der Atemzyklus des Patienten vorhergesagt werden, welches und/oder welcher während des ersten Atemzyklus zu erwarten wäre. Bei deren Vergleich kann das künstliche neuronale Netz besonders gut hinsichtlich des Patienten trainiert werden. Diese Ausführungsform des Trainings kann kontinuierlich mit dem Erfassen von Atemsignalen erfolgen. Die Prognose eines ersten Atemzyklus im Rahmen des Trainings kann analog zu einer Vorhersage eines zukünftigen Atemsignals erfolgen.

Ein derart trainiertes künstliches neuronales Netz kann insbesondere auf Atemsignalen mehrerer Personen und auf dem zeitaufgelösten Atemsignals von zumindest einem Atemzyklus des Patienten basieren. Dadurch kann es detailliert und gleichzeitig individuell ausgestaltet sein. Dadurch kann eine robuste Vorhersage eines zukünftigen Atemsignals gewährleistet werden.

Eine Ausführungsform des Verfahrens sieht vor, dass die Prognose eines ersten Atemzyklus des zumindest einen Atemzyklus des Patienten basierend auf dem künstlichen neuronalen Netz erfolgt. Dadurch kann eine robuste Vorhersage gewährleistet werden.

Eine Ausführungsform des Verfahrens sieht vor, dass das zukünftige Atemsignal für einen ersten Zeitpunkt eines folgenden Atemzyklus vorhergesagt wird. Der folgende Atemzyklus kann sich direkt an den zumindest einem Atemzyklus des Patienten anschließen, wessen zeitaufgelöstes Atemsignal erfasst wurde. Zwischen dem zumindest einen Atemzyklus des Patienten, wessen zeitaufgelöstes Atemsignal erfasst wurde und dem folgenden Atemzyklus können zumindest ein Atemzyklus, insbesondere zumindest zwei Atemzyklen, höchst insbesondere zumindest drei Atemzyklen vergehen. Das zukünftige Atemsignal wird vorzugsweise für zumindest einen ersten Zeitpunkt eines folgenden Atemzyklus vorhergesagt. Der erste Zeitpunkt korreliert vorzugsweise mit einem für die Triggerung relevanten Zeitpunkt. Diese Ausführungsform ermöglicht eine besonders robuste atmungsgetriggerte Aufnahme.

Eine Ausführungsform des Verfahrens sieht vor, dass das zukünftige Atemsignal zeitaufgelöst für einen folgenden Atemzyklus umfassend den ersten Zeitpunkt bestimmt wird. Gemäß dieser Ausführungsform wird das zukünftige Atemsignal für zumindest einen folgenden Atemzyklus zeitaufgelöst vorhergesagt. Insbesondere kann dadurch die Atmung besonders kontinuierlich vorhergesagt werden. Änderungen der zukünftigen Atmung können dabei besonders gut überwacht werden, wodurch der für die Triggerung relevante Zeitpunkt besonders genau vorhergesagt werden kann. Diese Ausführungsform ermöglicht eine besonders robuste atmungsgetriggerte Aufnahme.

Eine Ausführungsform des Verfahrens sieht vor, dass der erste Zeitpunkt durch eine Eigenschaft des Atemsignals charakterisiert ist. Der erste Zeitpunkt charakterisiert vorzugsweise den folgenden Atemzyklus durch das vorhergesagte zukünftige Atemsignal des Patienten zum ersten Zeitpunkt. Das vorhergesagte zukünftige Atemsignal des Patienten zum ersten Zeitpunkt weist vorzugsweise eine bestimmte Eigenschaft, insbesondere eine vorgegebene Charakteristik auf. Die Eigenschaft und/oder die Charakteristik kann beispielsweise einen Übergang vom Einatmen zum Ausatmen und/oder einen Übergang vom Ausatmen zum Einatmen umfassen. Die Eigenschaft und/oder die Charakteristik kann auch ein maximales und/oder minimales und/oder relatives maximales und/oder relatives minimales Lungenvolumen innerhalb des folgenden Atemzyklus umfassen. Das absolute maximale und/oder absolute minimale Lungenvolumen ist vorzugsweise irrelevant und/oder wird nicht bestimmt. Der erste Zeitpunkt ist vorzugsweise dadurch gegeben, dass zum ersten Zeitpunkt eine Eigenschaft und/oder Charakteristik des Atemzyklus erfüllt ist. Basierend auf dem ersten Zeitpunkt und/oder dem Atemsignal zum ersten Zeitpunkt erfolgt vorzugsweise die Triggerung der Aufnahme von medizinischen Rohdaten. Der erste Zeitpunkt für die Triggerung kann dadurch besonders genau bestimmt werden. Insbesondere kann die Triggerung individuell auf die aktuelle Atmung des Patienten abgestimmt werden. Dabei kann die aktuelle Atmung des Patienten zum für die Triggerung relevanten ersten Zeitpunkt besonders gut prospektiv bestimmt werden.

Eine Ausführungsform des Verfahrens sieht vor, dass die Triggerung der Aufnahme von medizinischen Rohdaten basierend auf dem ersten Zeitpunkt erfolgt.

Eine Ausführungsform des Verfahrens sieht vor, dass die Triggerung den Beginn der Aufnahme der medizinischen Rohdaten des Patienten bestimmt. Insbesondere bestimmt der erste Zeitpunkt den Beginn der Aufnahme der medizinischen Rohdaten des Patienten. Die Triggerung kann zeitgleich mit dem ersten Zeitpunkt erfolgen. Es kann zeitlich abhängig vom ersten Zeitpunkt der Beginn der Aufnahme der medizinischen Rohdaten bestimmt werden. Werden die medizinischen Rohdaten beispielsweise mit einem Magnetresonanzgerät mittels einer MR-Steuerungssequenz aufgenommen, so kann vor dem ersten Zeitpunkt, vorzugsweise zu einem Zeitpunkt definiert durch einen vorgegebenen Zeitabstand vor dem ersten Zeitpunkt, ein Präparations-Puls emittiert werden. Zum ersten Zeitpunkt und/oder zu einem definierten Zeitpunkt nach dem ersten Zeitpunkt kann die Akquisition der medizinischen Rohdaten beginnen. Dadurch können im Vergleich zu herkömmlichen Verfahren mehr medizinische Rohdaten im Rahmen einer Akquisition aufgenommen werden. Dadurch kann die Dauer für die Aufnahme der gesamten medizinischen Rohdaten reduziert und/oder die Qualität der medizinischen Rohdaten erhöht werden.

Zusätzlich und/oder alternativ kann die Triggerung ein Signal zur Atemanhaltung umfassen. Umfasst die Triggerung ein Signal zur Atemanhaltung, so kann der definierte Zeitpunkt nach dem ersten Zeitpunkt beispielsweise sicherstellen, dass der Patient den Atem bis zum definierten Zeitpunkt nach dem ersten Zeitpunkt tatsächlich anhält. Dies kann auch als Puffer bezeichnet werden. Diese Ausführungsform ermöglicht eine besonders effiziente Aufnahme der medizinischen Rohdaten. Die Triggerung kann dabei besonders gut auf die Atmung des Patienten abgestimmt werden.

Eine Ausführungsform des Verfahrens sieht vor, dass die Triggerung ein Signal an den Patienten zur Anhaltung der Atmung auslöst. Das Signal an den Patienten zur Anhaltung der Atmung wird vorzugsweise abhängig vom ersten Zeitpunkt ausgelöst. Das Signal an den Patienten zur Anhaltung der Atmung kann zum ersten Zeitpunkt ausgelöst werden und/oder die Akquisition der medizinischen Rohdaten kann zum ersten Zeitpunkt beginnen. Ebenso können das Signal an den Patienten zur Anhaltung der Atmung und der Beginn der Akquisition der medizinischen Rohdaten zeitlich versetzt sein. Dies kann die tatsächliche Anhaltung der Atmung zum Beginn der Akquisition der medizinischen Rohdaten sicherstellen. Das Signal kann beispielsweise mittels Sprache und/oder visuell und/oder mechanisch an den Patienten übermittelt werden. Diese Ausführungsform ermöglicht eine besonders exakte Triggerung. Insbesondere kann die Aufnahme medizinischer Rohdaten bei Anhaltung der Atmung ausgeführt werden, wobei der Zeitpunkt des Beginnes der Anhaltung der Atmung individuell auf den Patienten, insbesondere auf dessen Atemzyklus, abgestimmt werden kann.

Erfolgt gemäß einer Ausführungsform die Bestimmung des zukünftigen Atemsignals zeitaufgelöst für einen folgenden Atemzyklus umfassend den ersten Zeitpunkt, so kann der erste Zeitpunkt ein beliebiger Zeitpunkt während des folgenden Atemzyklus sein. Entsprechend kann gemäß dieser Ausführungsform auch ein beliebiger Zeitpunkt während des folgenden Atemzyklus zur Triggerung des Beginns der Aufnahme der medizinischen Rohdaten des Patienten herangezogen werden.

Des Weiteren geht die Erfindung aus von einem medizinischen Bildgebungsgerät mit einer Steuerungseinheit umfassend eine Aufnahmesteuereinheit mit einer Erfassungseinheit, einer Vorhersageeinheit und einer Triggereinheit. Die Steuerungseinheit, insbesondere die Aufnahmesteuereinheit, ist dazu ausgebildet, ein erfindungsgemäßes Verfahren zu einer atmungsgetriggerten Aufnahme von medizinischen Rohdaten auszuführen.

Die Erfassungseinheit ist typischerweise dazu ausgebildet, ein zeitaufgelöstes Atemsignal von zumindest einem Atemzyklus eines Patienten zu erfassen. Hierfür weist die Erfassungseinheit und/oder die Aufnahmesteuereinheit typischerweise einen Eingang, eine Prozessoreinheit und einen Ausgang auf. Die Erfassungseinheit kann dazu ausgebildet sein, ein zeitaufgelöstes Atemsignal eines Patienten aufzunehmen. Hierfür kann die Erfassungseinheit Sensoren wie beispielsweise eine Kamera und/oder Bewegungssensoren, welche vorzugsweise am Patienten anliegen, und/oder ein EKG umfassen. Über den Eingang der Erfassungseinheit kann ein zeitaufgelöstes Atemsignal bereitgestellt werden und/oder Parameter und/oder Software bereitgestellt werden, welche zum Erfassen des zeitaufgelöstes Atemsignals erforderlich sind. Über den Ausgang kann ein zeitaufgelöstes Atemsignal der Vorhersageeinheit und/oder weiteren für das erfindungsgemäße Verfahren benötigten Einheiten bereitgestellt werden.

Die Vorhersageeinheit ist typischerweise dazu ausgebildet, ein zukünftiges Atemsignal eines Patienten für einen folgenden Atemzyklus basierend auf dem zeitaufgelösten Atemsignal zu bestimmen. Hierfür weist die Vorhersageeinheit und/oder die Aufnahmesteuereinheit typischerweise einen Eingang, eine Prozessoreinheit und einen Ausgang auf. Über den Eingang können der Vorhersageeinheit ein zeitaufgelöstes Atemsignal bereitgestellt werden und/oder Parameter und/oder Software bereitgestellt werden, welche zur Vorhersage eines zukünftigen Atemsignals erforderlich sind. Weitere, im Verfahren benötigte Funktionen, Algorithmen oder Parameter können der Aufnahmesteuereinheit und/oder der Vorhersageeinheit über den Eingang bereitgestellt werden. Das zukünftige Atemsignal und/oder weitere Ergebnisse einer Vorhersage eines zukünftigen Atemsignals und/oder einer Ausführungsform des erfindungsgemäßen Verfahrens können über den Ausgang bereitgestellt werden.

Die Triggereinheit ist typischerweise dazu ausgebildet, eine Aufnahme von medizinischen Rohdaten des Patienten zu initiieren und/oder eine Aufnahme von medizinischen Rohdaten des Patienten auf das vorhergesagte zukünftige Atemsignal des Patienten abzustimmen. Hierfür weist die Triggereinheit und/oder die Aufnahmesteuereinheit typischerweise einen Eingang, eine Prozessoreinheit und einen Ausgang auf. Über den Eingang können der Triggereinheit ein zukünftiges Atemsignal des Patienten bereitgestellt werden und/oder Parameter und/oder Software bereitgestellt werden, welche zur Triggerung einer Aufnahme von medizinischen Rohdaten erforderlich sind. Weitere, im Verfahren benötigte Funktionen, Algorithmen oder Parameter können der Aufnahmesteuereinheit und/oder der Triggereinheit über den Eingang bereitgestellt werden. Ein Triggerimpuls und/oder Beginnzeitpunkt für die Aufnahme von medizinischen Rohdaten und/oder weitere Ergebnisse können über den Ausgang bereitgestellt werden.

Die Aufnahmesteuereinheit kann einen Eingang, eine Prozessoreinheit und einen Ausgang aufweisen, welche zumindest teilweise von der Erfassungseinheit und/oder der Vorhersageeinheit und/oder der Triggereinheit verwendet werden. Die Steuerungseinheit und/oder die Aufnahmesteuereinheit und/oder die Erfassungseinheit und/oder die Vorhersageeinheit und/oder die Triggereinheit können in das medizinische Bildgebungsgerät integriert sein. Die Steuerungseinheit und/oder die Aufnahmesteuereinheit und/oder die Erfassungseinheit und/oder die Vorhersageeinheit und/oder die Triggereinheit können auch separat von dem medizinischen Bildgebungsgerät installiert sein. Die Steuerungseinheit und/oder die Aufnahmesteuereinheit und/oder die Erfassungseinheit und/oder die Vorhersageeinheit und/oder die Triggereinheit können mit dem medizinischen Bildgebungsgerät verbunden sein. Die Aufnahmesteuereinheit und/oder die Erfassungseinheit und/oder die Vorhersageeinheit und/oder die Triggereinheit können von der Steuerungseinheit umfasst sein und/oder in diese integriert sein und/oder mit dieser verbunden sein. Die Aufnahmesteuereinheit und/oder die Erfassungseinheit und/oder die Vorhersageeinheit und/oder die Triggereinheit können auch separat von der Steuerungseinheit ausgebildet sein. Die Erfassungseinheit und/oder die Vorhersageeinheit und/oder die Triggereinheit können von der Aufnahmesteuereinheit umfasst sein und/oder in diese integriert sein und/oder mit dieser verbunden sein. Die Erfassungseinheit und/oder die Vorhersageeinheit und/oder die Triggereinheit können auch separat von der Aufnahmesteuereinheit ausgebildet sein.

Ausführungsformen des erfindungsgemäßen medizinischen Bildgebungsgerätes sind analog zu den Ausführungsformen des erfindungsgemäßen Verfahrens ausgebildet. Das medizinische Bildgebungsgerät kann weitere Steuerungskomponenten aufweisen, welche zum Ausführen eines erfindungsgemäßen Verfahrens nötig und/oder vorteilhaft sind. Auch kann das medizinische Bildgebungsgerät dazu ausgebildet sein, Steuerungssignale zu senden und/oder Steuerungssignale zu empfangen und/oder zu verarbeiten, um ein erfindungsgemäßes Verfahren auszuführen. Vorzugsweise ist die Erfassungseinheit und/oder die Vorhersageeinheit und/oder die Triggereinheit Teil der Aufnahmesteuereinheit und/oder der Steuerungseinheit des erfindungsgemäßen medizinischen Bildgebungsgeräts. Auf einer Speichereinheit der Aufnahmesteuereinheit können Computerprogramme und weitere Software gespeichert sein, mittels derer die Prozessoreinheit der Aufnahmesteuereinheit einen Verfahrensablauf eines erfindungsgemäßen Verfahrens automatisch steuert und/oder ausführt.

Ein erfindungsgemäßes Computerprogrammprodukt ist direkt in einer Speichereinheit einer programmierbaren Aufnahmesteuereinheit ladbar und weist Programmcode-Mittel auf, um ein erfindungsgemäßes Verfahren auszuführen, wenn das Computerprogrammprodukt in der Aufnahmesteuereinheit ausgeführt wird. Dadurch kann das erfindungsgemäße Verfahren schnell, identisch wiederholbar und robust ausgeführt werden. Das Computerprogrammprodukt ist so konfiguriert, dass es mittels der Aufnahmesteuereinheit die erfindungsgemäßen Verfahrensschritte ausführen kann. Die Aufnahmesteuereinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem elektronisch lesbaren Medium gespeichert oder auf einem Netzwerk oder Server hinterlegt, von wo es in den Prozessor einer lokalen Aufnahmesteuereinheit geladen werden kann, der mit dem medizinischen Bildgebungsgerät direkt verbunden oder als Teil des medizinischen Bildgebungsgeräts ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Aufnahmesteuereinheit eines medizinischen Bildgebungsgeräts ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software, gespeichert ist. Wenn diese Steuerinformationen (Software) von dem Datenträger gelesen und in eine Aufnahmesteuereinheit und/oder Erfassungseinheit und/oder Vorhersageeinheit und/oder Triggereinheit eines medizinischen Bildgebungsgeräts gespeichert werden, können alle erfindungsgemäßen Ausführungsformen der vorab beschriebenen Verfahren durchgeführt werden.

Des Weiteren geht die Erfindung aus von einem elektronisch lesbaren Datenträger, auf dem ein Programm hinterlegt ist, das zu einer Ausführung eines Verfahrens zu einer atmungsgetriggerten Aufnahme von medizinischen Rohdaten, vorgesehen ist.

Die Vorteile des erfindungsgemäßen medizinischen Bildgebungsgeräts, des erfindungsgemäßen Computerprogrammprodukts und des erfindungsgemäßen elektronisch lesbaren Datenträgers entsprechen im Wesentlichen den Vorteilen des erfindungsgemäßen Verfahrens zu einer atmungsgetriggerten Aufnahme von medizinischen Rohdaten, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein erfindungsgemäßes medizinisches Bildgebungsgerät in einer schematischen Darstellung,
- Fig. 2: ein Ablaufdiagramm einer ersten Ausführungsform eines erfindungsgemäßen Verfahrens,
- Fig. 3: ein Ablaufdiagramm einer zweiten Ausführungsform eines erfindungsgemäßen Verfahrens,
- Fig. 4: ein Ablaufdiagramm einer dritten Ausführungsform eines erfindungsgemäßen Verfahrens,
- Fig. 5: ein zeitaufgelöstes Atemsignal und ein zukünftiges Atemsignal in einer schematischen Darstellung.

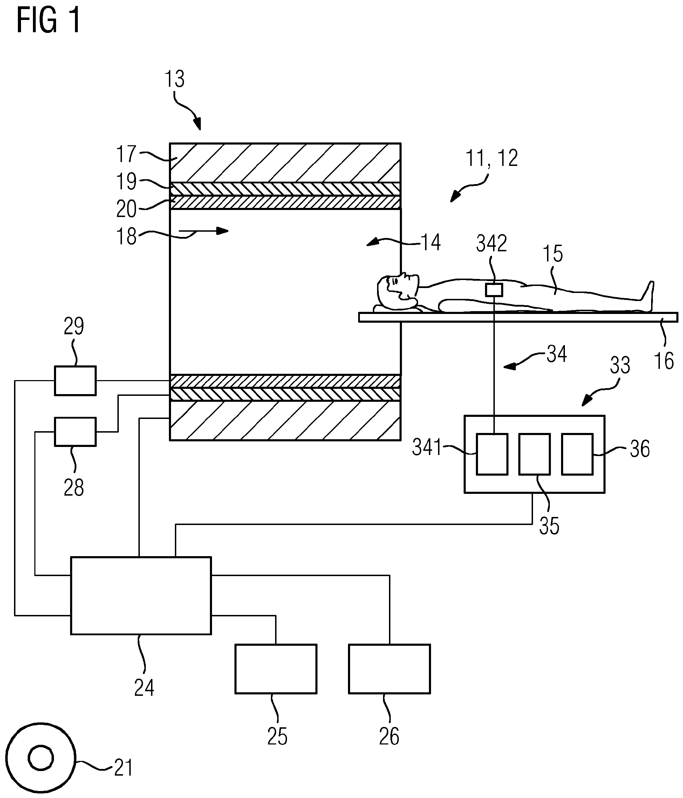

Figur 1 zeigt ein Magnetresonanzgerät 11 als Beispiel für ein erfindungsgemäßes medizinisches Bildgebungsgerät 12 zur Ausführung eines erfindungsgemäßen Verfahrens in einer schematischen Darstellung. Grundsätzlich ist die Ausgestaltung des medizinischen Bildgebungsgerätes 12 nicht auf ein Magnetresonanzgerät 11 eingeschränkt, sondern kann auch von weiteren, dem Fachmann als sinnvoll erscheinenden medizinischen Bildgebungsgeräten 12, wie beispielsweise Computertomographen, PET, Ultraschallgeräten, usw. gebildet sein.

Das Magnetresonanzgerät 11 umfasst eine von einer Magneteinheit 13 gebildeten Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Patienten 15 auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Patiententisch auf, der bewegbar innerhalb des Magnetresonanzgeräts 11 angeordnet ist.

Die Magneteinheit 13 weist weiterhin eine Gradientenspuleneinheit 19 auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Hochfrequenz-Pulse in einen Untersuchungsraum, der im Wesentlichen von dem Patientenaufnahmebereich 14 gebildet ist, ein.

Zu einer Steuerung des Hauptmagneten 17, der Gradientensteuereinheit 28 und der Hochfrequenzantennensteuereinheit 29 weist das Magnetresonanzgerät 11 eine Steuerungseinheit 24 auf. Die Steuerungseinheit 24 steuert zentral das Magnetresonanzgerät 11, wie beispielsweise das Durchführen von MR-Steuerungssequenzen. Zudem umfasst die Steuerungseinheit 24 eine nicht näher dargestellte Rekonstruktionseinheit zu einer Rekonstruktion von Bilddaten aus medizinischen Rohdaten, die während der Magnetresonanzuntersuchung erfasst werden. Das Magnetresonanzgerät 11 weist eine Anzeigeeinheit 25 auf. Steuerinformationen wie beispielsweise Steuerungsparameter, sowie rekonstruierte Bilddaten können auf der Anzeigeeinheit 25, beispielsweise auf zumindest einem Monitor, für einen Benutzer angezeigt werden. Zudem weist das Magnetresonanzgerät 11 eine Eingabeeinheit 26 auf, mittels derer Informationen und/oder Steuerungsparameter während eines Messvorgangs von einem Benutzer eingegeben werden können. Die Steuerungseinheit 24 kann die Gradientensteuereinheit 28 und/oder Hochfrequenzantennensteuereinheit 29 und/oder die Anzeigeeinheit 25 und/oder die Eingabeeinheit 26 umfassen.

Das medizinische Bildgebungsgerät umfasst weiterhin eine Aufnahmesteuereinheit 33 mit einer Erfassungseinheit 34, einer Vorhersageeinheit 35 und einer Triggereinheit 36. Die Erfassungseinheit 34 umfasst in der dargestellten Ausführungsform einen Erfassungssensor 342 und eine Erfassungsbestimmungseinheit 341. Der Erfassungssensor 342 ist vorzugsweise dazu ausgebildet, Daten hinsichtlich einer Bewegung und/oder einer Atmung des Patienten 15 aufzunehmen und der Erfassungsbestimmungseinheit 341 bereitzustellen. Die Erfassungsbestimmungseinheit 341 ist vorzugsweise dazu ausgebildet, basierend auf den Daten ein zeitaufgelöstes Atemsignal zu ermitteln. Die Erfassungseinheit 34 kann auch ohne Erfassungssensor 342 und/oder ohne Erfassungsbestimmungseinheit 341 ausgebildet sein. Die Erfassungseinheit 34 kann mit einer weiteren Einheit verbunden sein, welche der Erfassungseinheit 34 Daten hinsichtlich einer Bewegung und/oder einer Atmung des Patienten 15 bereitstellt, basierend auf welchen die Erfassungseinheit 34 ein zeitaufgelöstes Atemsignal generiert. Die Erfassungseinheit 34 kann mit einer weiteren Einheit verbunden sein, welche der Erfassungseinheit 34 ein zeitaufgelöstes Atemsignal bereitstellt.

Die Aufnahmesteuereinheit 33 ist zu einer Ausführung eines Verfahrens zu einer atmungsgetriggerten Aufnahme von medizinischen Rohdaten ausgelegt. Hierzu weist die Aufnahmesteuereinheit 33 Computerprogramme und/oder Software auf, die direkt in einem nicht näher dargestellten Speichereinheit der Aufnahmesteuereinheit 33 ladbar sind, mit Programmmitteln, um ein Verfahren zu einer atmungsgetriggerten Aufnahme von medizinischen Rohdaten auszuführen, wenn die Computerprogramme und/oder Software in der Aufnahmesteuereinheit 33 ausgeführt werden. Die Aufnahmesteuereinheit 33 weist hierzu einen nicht näher dargestellten Prozessor auf, der zu einer Ausführung der Computerprogramme und/oder Software ausgelegt ist. Die Aufnahmesteuereinheit 33 kann wie im dargestellten Fall separat von der Steuerungseinheit 24 ausgebildet sein. Ebenso ist denkbar, dass die Aufnahmesteuereinheit 33 in die Steuerungseinheit 24 ist. Die Aufnahmesteuereinheit 33 kann zumindest teilweise in die Steuerungseinheit 24 integriert sein. Beispielsweise kann die Vorhersageeinheit 35 und die Triggereinheit 36 von der Steuerungseinheit 24 umfasst werden. Die Erfassungseinheit 34 kann zumindest teilweise separat von der Steuerungseinheit 24 ausgebildet sein. Beispielsweise kann die Erfassungsbestimmungseinheit 341 in die Steuerungseinheit 24 sein und der Erfassungssensor 342 separat von der Steuerungseinheit 24 ausgebildet sein.

Alternativ hierzu können die Computerprogramme und/oder Software auch auf einem getrennt von der Steuerungseinheit 24 und/oder Aufnahmesteuereinheit 33 ausgebildeten elektronisch lesbaren Datenträger 21 gespeichert sein, wobei ein Datenzugriff von der Aufnahmesteuereinheit 33 auf den elektronisch lesbaren Datenträger 21 über ein Datennetz erfolgen kann.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird. Das Magnetresonanzgerät 11 ist somit zusammen mit der Aufnahmesteuereinheit 33 zur Ausführung eines erfindungsgemäßen Verfahrens ausgelegt.

Ein Verfahren zu einer atmungsgetriggerten Aufnahme von medizinischen Rohdaten kann auch in Form eines Computerprogrammprodukts vorliegen, das das Verfahren auf die Aufnahmesteuereinheit 33 implementiert, wenn es auf der Aufnahmesteuereinheit 33 ausgeführt wird. Ebenso kann ein elektronisch lesbarer Datenträger 21 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 21 in einer Aufnahmesteuereinheit 33 eines Magnetresonanzgeräts 11 das beschriebene Verfahren durchführen.

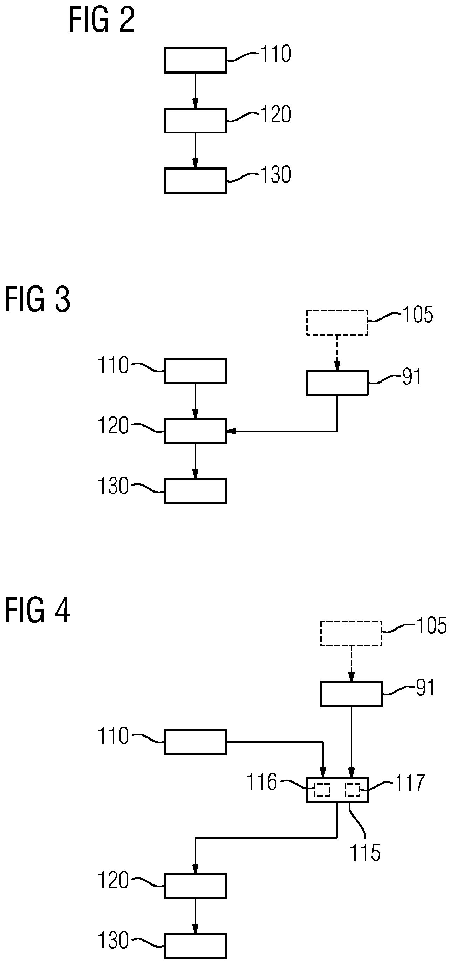

Figur 2 zeigt ein Ablaufdiagramm einer ersten Ausführungsform eines erfindungsgemäßen Verfahrens zu einer atmungsgetriggerten Aufnahme von medizinischen Rohdaten eines Patienten 15.

Gemäß Verfahrensschritt 110 erfolgt das Erfassen eines zeitaufgelösten Atemsignals von zumindest einem Atemzyklus des Patienten 15. In Verfahrensschritt 120 erfolgt die Vorhersage eines zukünftigen Atemsignals des Patienten 15 für einen folgenden Atemzyklus basierend auf dem zeitaufgelösten Atemsignal. Anschließend erfolgt in Verfahrensschritt 130 die Triggerung einer Aufnahme von medizinischen Rohdaten des Patienten 15 basierend auf dem vorhergesagten zukünftigen Atemsignal.

Figur 3 zeigt ein Ablaufdiagramm einer zweiten Ausführungsform eines erfindungsgemäßen Verfahrens zu einer atmungsgetriggerten Aufnahme von medizinischen Rohdaten eines Patienten 15. Gemäß dieser Ausführungsform erfolgt die Vorhersage des zukünftigen Atemsignals gemäß Verfahrensschritt 120 auf basierend auf einem künstlichen neuronalen Netz 91. Dieses künstliche neuronale Netz 91 kann optional in einem vorhergehenden Verfahrensschritt 105 basierend auf Atemsignalen mehrerer Personen trainiert worden sein.

Figur 4 zeigt ein Ablaufdiagramm einer dritten Ausführungsform eines erfindungsgemäßen Verfahrens zu einer atmungsgetriggerten Aufnahme von medizinischen Rohdaten eines Patienten 15. Gemäß dieser Ausführungsform erfolgt in einem weiteren Verfahrensschritt 115 ein Training des künstlichen neuronalen Netzes 91 basierend auf dem in Verfahrensschritt 110 erfassten zeitaufgelösten Atemsignal des zumindest einen Atemzyklus des Patienten 15. Die Vorhersage des zukünftigen Atemsignals in Verfahrensschritt 120 erfolgt gemäß dieser Ausführungsform basierend auf dem trainierten künstlichen neuronalen Netz. Bei der Vorhersage des zukünftigen Atemsignals in Verfahrensschritt 120 kann optional zusätzlich direkt das in Verfahrensschritt 110 erfasste zeitaufgelöste Atemsignal des zumindest einen Atemzyklus des Patienten 15 berücksichtigt werden. Das Training in Verfahrensschritt 115 kann eine Prognose 116 eines ersten Atemzyklus des zumindest einen Atemzyklus des Patienten 15 und einen Vergleich 117 des vorhergesagten Atemzyklus mit dem ersten Atemzyklus des zumindest einen Atemzyklus umfassen. Die Prognose 116 kann dabei basierend auf dem künstlichen neuronalen Netz 91 erfolgen.

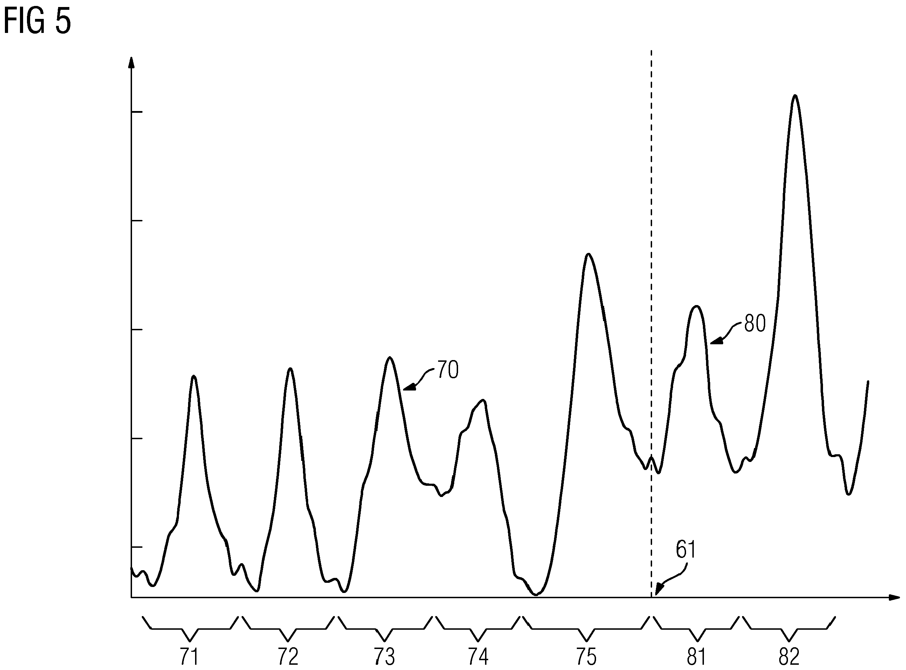

Figur 5 zeigt ein zeitaufgelöstes Atemsignal 70 und ein vorhergesagtes zukünftiges Atemsignal 80 in einer schematischen Darstellung. Die horizontale Richtung stellt den Zeitverlauf, die vertikale Richtung ein Maß für das Atemsignal, wie beispielsweise eine Bewegung des Brustkörpers des Patienten 15, dar. Das Atemsignal 70 wurde im visualisierten Fall für zumindest fünf Atemzyklen 71, 72, 73, 74, 75 erfasst. Das zukünftige Atemsignal 80 wird gemäß einer Ausführungsform des erfindungsgemäßen Verfahrens für zumindest einen ersten Zeitpunkt 61 eines folgenden Atemzyklus 81, 82 vorhergesagt. Der erste Zeitpunkt 61 kann dabei von einem direkt auf den letzten erfassten Atemzyklus 75 folgenden Atemzyklus 81 umfasst sein. Der erste Zeitpunkt 61 kann auch von einem auf den letzten erfassten Atemzyklus 75 später folgenden Atemzyklus 82 umfasst sein. Das zukünftige Atemsignal 80 kann auch für mehrere Zeitpunkte eines und/oder mehrerer folgender Atemzyklen 81, 82 gemäß einer Ausführungsform des erfindungsgemäßen Verfahrens bestimmt werden. Insbesondere kann das zukünftige Atemsignal 80 zeitaufgelöst für zumindest einen folgenden Atemzyklus 81, 82 bestimmt werden. Der zumindest eine folgende Atemzyklus 81, 82 umfasst dabei typischerweise den ersten Zeitpunkt 61.

Der erste Zeitpunkt 61 ist vorzugsweise durch eine Eigenschaft des Atemsignals, insbesondere des zukünftigen Atemsignals 80, charakterisiert. Im dargestellten Fall ist der erste Zeitpunkt 61 beispielsweise durch die Änderung der Atmung des Patienten 15 von "Ausatmen" zu "Einatmen" und/oder durch ein im Atemzyklus 81 minimales Volumen der Luft in der Lunge des Patienten vor dem Einatmen innerhalb des Atemzyklus 81 gekennzeichnet. Die Triggerung der Aufnahme von medizinischen Rohdaten erfolgt vorzugsweise basierend auf dem ersten Zeitpunkt 61. Insbesondere kann der erste Zeitpunkt 61 und/oder die Triggerung den Beginn der Aufnahme der medizinischen Rohdaten des Patienten 15 bestimmen. Ebenso kann abhängig vom ersten Zeitpunkt 61 ein Signal an den Patienten 15 zur Anhaltung der Atmung ausgelöst werden.
Die Triggerung kann ein Signal an den Patienten 15 zur Anhaltung der Atmung, vorzugsweise abhängig vom ersten Zeitpunkt 61, auslösen. Der erste Zeitpunkt 61 kann ein beliebiger Zeitpunkt des vorhergesagten zukünftigen Atemsignals 80 sein.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer atmungsgetriggerten Aufnahme von medizinischen Rohdaten eines Patienten umfassend die folgenden Verfahrensschritte:
- Erfassen eines zeitaufgelösten Atemsignals von zumindest einem Atemzyklus des Patienten,
- Vorhersage eines zukünftigen Atemsignals des Patienten basierend auf dem zeitaufgelösten Atemsignal,
- Triggerung einer Aufnahme von medizinischen Rohdaten des Patienten basierend auf dem vorhergesagten zukünftigen Atemsignal.

2. Verfahren nach Anspruch 1
wobei die Vorhersage des zukünftigen Atemsignals basierend auf einem künstlichen neuronalen Netz erfolgt.

3. Verfahren nach Anspruch 2,
wobei das künstliche neuronale Netz basierend auf Atemsignalen mehrerer Personen trainiert vorliegt.

4. Verfahren nach Anspruch 2 oder 3,
wobei ein weiterer Verfahrensschritt ein Training des künstlichen neuronalen Netzes basierend auf dem zeitaufgelösten Atemsignal des zumindest einen Atemzyklus des Patienten umfasst und die Vorhersage des zukünftigen Atemsignals basierend auf dem trainierten künstlichen neuronalen Netz erfolgt.

5. Verfahren nach Anspruch 4,
wobei das Training eine Prognose eines ersten Atemzyklus des zumindest einen Atemzyklus des Patienten und einen Vergleich des vorhergesagten Atemzyklus mit dem ersten Atemzyklus des zumindest einen Atemzyklus umfasst.

6. Verfahren nach Anspruch 5,
wobei die Prognose eines ersten Atemzyklus des zumindest einen Atemzyklus des Patienten basierend auf dem künstlichen neuronalen Netz erfolgt.

7. Verfahren nach einem der vorangehenden Ansprüche,
wobei das zukünftige Atemsignal für einen ersten Zeitpunkt eines folgenden Atemzyklus vorhergesagt wird.

8. Verfahren nach Anspruch 7,
wobei das zukünftige Atemsignal zeitaufgelöst für einen folgenden Atemzyklus umfassend den ersten Zeitpunkt bestimmt wird.

9. Verfahren nach einem der Ansprüche 7 bis 8,
wobei der erste Zeitpunkt durch eine Eigenschaft des Atemsignals charakterisiert ist.

10. Verfahren nach einem der Ansprüche 7 bis 9,
wobei die Triggerung der Aufnahme von medizinischen Rohdaten basierend auf dem ersten Zeitpunkt erfolgt.

11. Verfahren nach einem der vorangehenden Ansprüche,
wobei die Triggerung den Beginn der Aufnahme der medizinischen Rohdaten des Patienten bestimmt.

12. Verfahren nach einem der vorangehenden Ansprüche,
wobei die Triggerung ein Signal an den Patienten zur Anhaltung der Atmung auslöst.

13. Medizinisches Bildgebungsgerät mit einer Steuerungseinheit umfassend eine Aufnahmesteuereinheit mit einer Erfassungseinheit, einer Vorhersageeinheit und einer Triggereinheit, die zu einer Ausführung eines Verfahrens zu einer atmungsgetriggerten Aufnahme von medizinischen Rohdaten nach einem der vorangehenden Ansprüche ausgelegt ist.

14. Computerprogrammprodukt, welches ein Programm umfasst und direkt in einen Speicher einer programmierbaren Aufnahmesteuereinheit ladbar ist, mit Programmmitteln, um ein Verfahren zu einer atmungsgetriggerten Aufnahme von medizinischen Rohdaten nach einem der Ansprüche 1 bis 12 auszuführen, wenn das Programm in der Aufnahmesteuereinheit ausgeführt wird.

15. Elektronisch lesbarer Datenträger, auf dem ein Programm hinterlegt ist, das derart ausgestaltet ist, dass das Programm bei Verwendung des Datenträgers in einer Aufnahmesteuereinheit das Verfahren zu einer atmungsgetriggerten Aufnahme von medizinischen Rohdaten nach einem der Ansprüche 1 bis 12 durchführt.
